# EUROPEAN PATENT APPLICATION

(11) **EP 4 417 135 A1**
(43) Date of publication of application: **21.08.2024**
(21) Application number: 21960596.1
(22) Date of filing: 13.10.2021
(51) Int. Cl.: A61B 8/00

(54) **ULTRASOUND DIAGNOSTIC SYSTEM AND MONITORING METHOD THEREFOR**

(71) Applicant: Fuji Corporation, Chiryu-shi, Aichi 472-8686 (JP)
(72) Inventor: YAMASHITA Yasuhiro, Chiryu-shi, Aichi 472-8686 (JP); YOSHIDA Naofumi, Chiryu-shi, Aichi 472-8686 (JP); SHIRAKAWA Yoshihiro, Chiryu-shi, Aichi 472-8686 (JP); ISHIKAWA So, Chiryu-shi, Aichi 472-8686 (JP); KONDO Shinya, Chiryu-shi, Aichi 472-8686 (JP); YOSHIMURA Masataka, Chiryu-shi, Aichi 472-8686 (JP)
(74) Representative: Grünecker Patent- und Rechtsanwälte PartG mbB
(86) International application number: PCT/JP2021/037822
(87) International publication number: WO 2023/062737

(57) **Abstract**

An ultrasound diagnostic system is an ultrasound diagnostic system that performs diagnosis by moving an ultrasound probe by a robot. The system includes an arm section configured to hold the ultrasound probe, a motor section configured to move the arm section, an amplifier section configured to supply power to the motor section, a robot control section configured to output a control signal to the amplifier section, and a monitoring section configured to monitor states of respective sections constituting a system and shift to a predetermined safety state when an abnormality occurs in any of the respective sections.

## Description

### Technical Field

The present description discloses an ultrasound diagnostic system and a monitoring method therefor.

### Background Art

Conventionally, an ultrasound diagnostic system, which includes a robot arm that holds an ultrasound probe and moves the ultrasound probe along a body surface of a subject, a storage section that stores instruction trajectory information for moving the ultrasound probe by the robot arm, and a control section that controls driving of the robot arm such that the ultrasound probe is moved according to the stored instruction movement trajectory, has been proposed as an ultrasound diagnostic system of this type (for example, see Patent Literature 1).

### Patent Literature

Patent Literature 1: JP-A-2017-159027

### Summary of the Invention

### Technical Problem

In the above-described system, an ultrasound probe can be moved in a stable trajectory by moving a robot arm according to trajectory information stored in advance, but no response is described with respect to a case where abnormality occurs in each section constituting the system.

A main object of the present disclosure is to appropriately respond to abnormality of each section constituting an ultrasound diagnostic system that performs diagnosis by moving an ultrasound probe by a robot.

### Solution to Problem

The present disclosure employs a following device to achieve the main object described above.

An ultrasound diagnostic system of the present disclosure for performing diagnosis by moving an ultrasound probe by a robot includes an arm section configured to hold the ultrasound probe, a motor section configured to move the arm section, an amplifier section configured to supply power to the motor section, a robot control section configured to output a control signal to the amplifier section, and a monitoring section configured to monitor states of respective sections constituting a system and shift to a predetermined safety state when an abnormality occurs in any of the respective sections.

An ultrasound diagnostic system of the present disclosure monitors states of respective sections constituting a system including an arm section, a motor section, an amplifier section, and a robot control section, and is shifted to a predetermined safety state when an abnormality occurs in any of the respective sections. Thereby, it is possible to perform ultrasound diagnosis while ensuring safety.

### Brief Description of Drawings

Fig. 1 is an external perspective view of an ultrasound diagnostic system including a robot device of the present embodiment.
Fig. 2 is a side view of a robot device.
Fig. 3 is a block diagram illustrating an electrical connection relationship between a robot device and an ultrasound diagnostic device.
Fig. 4 is a flowchart illustrating an example of assist control processing.
Fig. 5 is an explanatory diagram illustrating an example of an operable area.
Fig. 6 is an explanatory diagram illustrating an example of a probe movement trajectory display processing.
Fig. 7A is an explanatory diagram illustrating a front-left hand mode.
Fig. 7B is an explanatory diagram illustrating a rear-left hand mode.
Fig. 7C is an explanatory diagram illustrating a front-right hand mode.
Fig. 7D is an explanatory diagram illustrating a rear-right hand mode.
Fig. 8A is an explanatory diagram illustrating a display viewpoint of a probe movement trajectory in a front-left hand mode and a rear-right hand mode.
Fig. 8B is an explanatory diagram illustrating a display viewpoint of a probe movement trajectory in a rear-left hand mode and a front-right hand mode.
Fig. 9 is a flowchart illustrating an example of monitoring.
Fig. 10 is an explanatory diagram illustrating monitoring function names and action due to abnormality.

### Description of Embodiments

Next, an embodiment for practicing the present disclosure will be described with reference to the drawings.

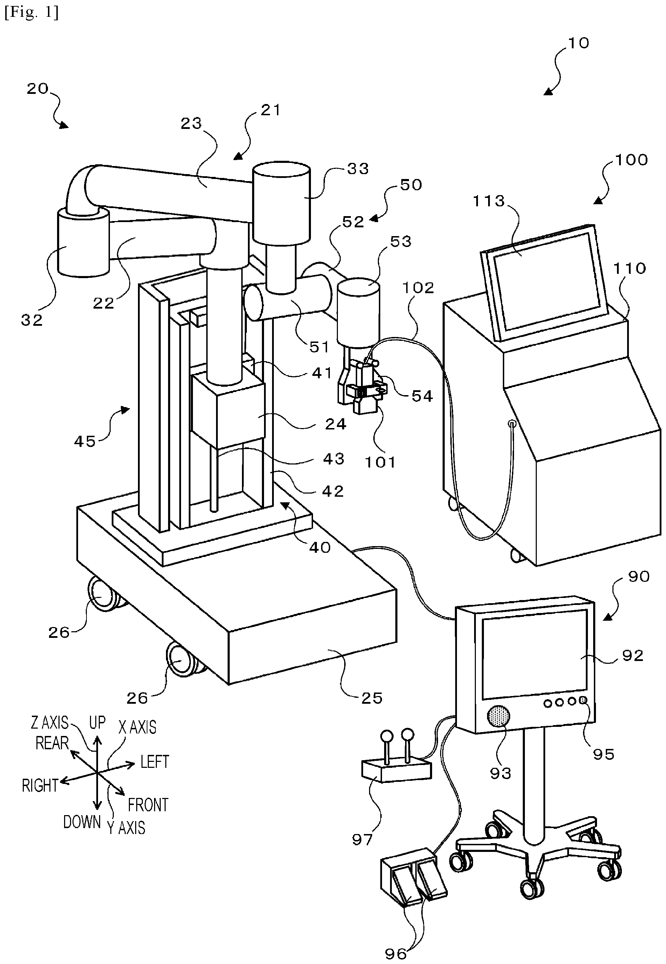

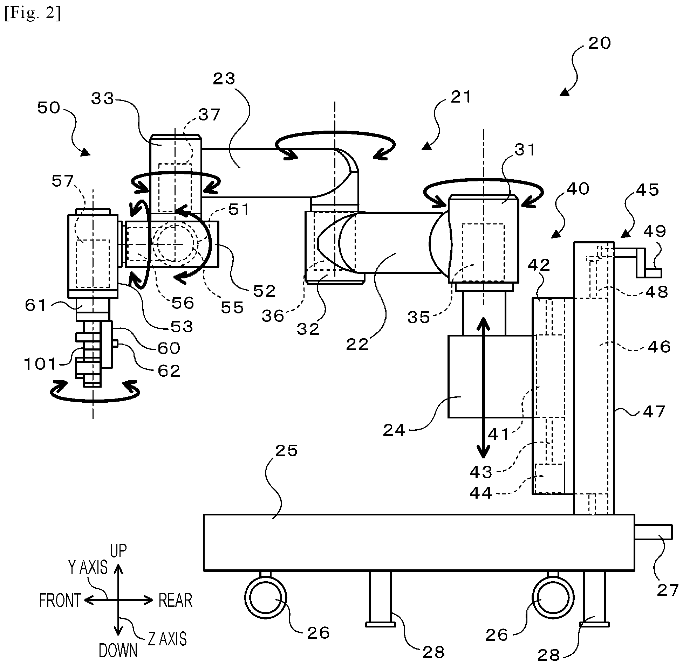

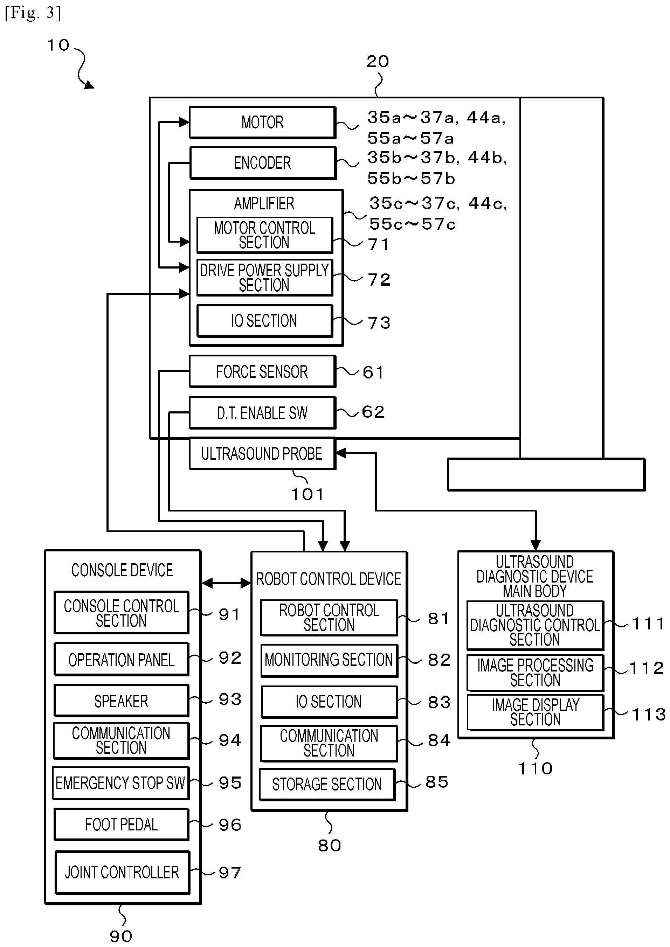

Fig. 1 is an external perspective view of ultrasound diagnostic system 10 including robot device 20 of the present embodiment. Fig. 2 is a side view of robot device 20. Fig. 3 is a block diagram illustrating an electrical connection relationship between robot device 20 and ultrasound diagnostic device 100. In Fig. 1, a left-right direction is an X axis, a front-rear direction is a Y-axis direction, and an up-down direction is a Z-axis direction.

Ultrasound diagnostic system 10 of the present embodiment obtains an ultrasound echo image by holding ultrasound probe 101 on a hand of robot arm 21 and operating robot device 20 such that ultrasound probe 101 is pressed against a body surface of a patient. Ultrasound diagnostic system 10 is used in, for example, catheter treatment. An operator (technician) who operates a guide wire of a catheter can press ultrasound probe 101 against a body surface of an examinee (patient) and moves the guide wire forward while recognizing a positional relationship between a front end of the guide wire and a blood vessel from the obtained ultrasound echo image, thereby causing the guide wire to accurately pass through the center of an occluded portion or a stenosed portion of the blood vessel.

As illustrated in Figs. 1 to 3, ultrasound diagnostic system 10 of the present embodiment includes robot device 20, console device 90, and ultrasound diagnostic device 100.

As illustrated in Fig. 1, ultrasound diagnostic device 100 includes ultrasound probe 101 and ultrasound diagnostic device main body 110 coupled to ultrasound probe 101 through cable 102. As illustrated in Fig. 3, ultrasound diagnostic device main body 110 includes ultrasound diagnostic control section 111 that controls the entire device, image processing section 112 that processes a reception signal from ultrasound probe 101 to generate an ultrasound echo image, image display section 113 that displays the ultrasound echo image, and various operation switches (not illustrated).

Console device 90 is provided separately from robot device 20, instructs various operations of robot device 20, and displays various types of information including a state of robot device 20. Console device 90 includes console control section 91 that controls the entire device, operation panel 92 that displays various types of information and can be touch-operated by an operator, speaker 93, communication section 94 that communicates with robot device 20, and emergency stop switch 95. Emergency stop switch 95 is a switch for stopping an operation of robot device 20 in an emergency. Furthermore, console device 90 is coupled to foot pedal 96 and joint controller 97 through cables. Foot pedal 96 and joint controller 97 are operation members capable of executing various instructions.

As illustrated in Figs. 1 to 3, robot device 20 includes base plate 25, robot arm 21 installed on base plate 25, height adjustment mechanism 45 that adjusts a height of robot arm 21 by a manual operation, and robot control device 80 that controls robot arm 21.

As illustrated in Figs. 1 and 2, casters 26 respectively having stoppers are attached to four corners of a rear surface of base plate 25. Robot device 20 can be moved more freely by casters 26. Further, at multiple locations (for example, three locations) on the rear surface of base plate 25, lock sections 28 are provided which protrude vertically downward by pushing down lever 27 and lock (fix) robot device 20 so as not to be movable.

As illustrated in Fig. 1, robot arm 21 includes first arm 22, second arm 23, base 24, first arm drive device 35, second arm drive device 36, posture holding device 37, and rotation three-axis mechanism 50.

A base end portion of first arm 22 is coupled to base 24 through first joint shaft 31 extending in an up-down direction (the Z-axis direction). First arm drive device 35 includes motor 35a, encoder 35b, and amplifier 35c. A rotation shaft of motor 35a is coupled to first joint shaft 31 through a decelerator (not illustrated). First arm drive device 35 rotates (revolves) first arm 22 along a horizontal plane (XY plane) around first joint shaft 31 as motor 35a rotationally drives first joint shaft 31. Encoder 35b is attached to a rotation shaft of motor 35a and is configured as a rotary encoder that detects a rotational displacement amount of motor 35a. Amplifier 35c is a drive section for driving motor 35a by switching of a switching element.

A base end portion of second arm 23 is coupled to a front end portion of first arm 22 through second joint shaft 32 extending in the up-down direction. Second arm drive device 36 includes motor 36a, encoder 36b, and amplifier 36c. A rotation shaft of motor 36a is coupled to second joint shaft 32 through a decelerator (not illustrated). Second arm drive device 36 rotates (revolves) second arm 23 along a horizontal plane around second joint shaft 32 as motor 36a rotationally drives second joint shaft 32. Encoder 36b is attached to the rotation shaft of motor 36a and is configured as a rotary encoder that detects a rotational displacement amount of motor 36a. Amplifier 35c is a drive section for driving motor 35a by switching of a switching element.

In the present embodiment, first arm 22 and second arm 23 constitute a horizontal joint arm. Therefore, robot device 20 has a right hand mode in which robot arm 21 operates in a right hand system and a left hand mode in which robot arm 21 operates in a left hand system as an operation mode.

Base 24 is provided to be lifted and lowered with respect to base plate 25 by lifting and lowering device 40 installed on base plate 25. As illustrated in Figs. 1 and 2, lifting and lowering device 40 includes first slider 41 fixed to base 24, first guide member 42 extending in the up-down direction and guiding the movement of first slider 41, first ball screw shaft 43 (a lifting and lowering shaft) extending in the up-down direction and screwed into a ball screw nut (not illustrated) fixed to first slider 41, motor 44a for rotationally driving first ball screw shaft 43, encoder 44b (see Fig. 3), and amplifier 44c for driving motor 44a. Lifting and lowering device 40 moves base 24 fixed to first slider 41 in the up-down direction along first guide member 42 by rotationally driving first ball screw shaft 43 by using motor 44a. Encoder 44b is configured as a linear encoder that detects a position (a lifting and lowering position) of first slider 41 (base 24) in the up-down direction.

As illustrated in Fig. 2, height adjustment mechanism 45 includes second slider 46 fixed to first guide member 42 of lifting and lowering device 40, second guide member 47 fixed to base plate 25 and extending in the up-down direction to guide the movement of second slider 46, second ball screw shaft 48 (a lifting and lowering shaft) extending in the up-down direction and screwed into a ball screw nut (not illustrated) fixed to second slider 46, and operation handle 49 coupled to second ball screw shaft 48 through a power transfer mechanism (a bevel gear). Height adjustment mechanism 45 moves first guide member 42 of lifting and lowering device 40 fixed to second slider 46 up and down along second guide member 47 by rotationally driving second ball screw shaft 48 by a manual operation of operation handle 49. A base end of robot arm 21 is fixed to base 24, and base 24 is supported by first guide member 42, and accordingly, a height of robot arm 21 can be adjusted by moving first guide member 42 up and down by using height adjustment mechanism 45. Thereby, the height of robot arm 21 can be adjusted according to a height of a bed on which, for example, an examinee of an ultrasound diagnosis lies.

As illustrated in Figs. 1 and 2, rotation three-axis mechanism 50 is coupled to a front end portion of second arm 23 through posture holding shaft 33 extending in the up-down direction. Rotation three-axis mechanism 50 includes first rotation shaft 51, second rotation shaft 52, and third rotation shaft 53 that are orthogonal to each other, first rotation device 55 that rotates first rotation shaft 51, second rotation device 56 that rotates second rotation shaft 52, and third rotation device 57 that rotates third rotation shaft 53. First rotation shaft 51 is supported in a posture orthogonal to posture holding shaft 33. Second rotation shaft 52 is supported in a posture orthogonal to first rotation shaft 51. Third rotation shaft 53 is supported in a posture orthogonal to second rotation shaft 52. First rotation device 55 includes motor 55a that rotationally drives first rotation shaft 51, encoder 55b that is attached to a rotation shaft of motor 55a and detects a rotational displacement amount of motor 55a, and amplifier 55c that drives motor 55a. Second rotation device 56 includes motor 56a that rotationally drives second rotation shaft 52, encoder 56b that is attached to a rotation shaft of motor 56a and detects a rotational displacement amount of motor 56a, and amplifier 56c that drives motor 56a. Third rotation device 57 includes motor 57a that rotationally drives third rotation shaft 53, encoder 57b that is attached to a rotation shaft of motor 57a and detects a rotational displacement amount of motor 57a, and amplifier 57c that drives motor 56a. Further, third rotation shaft 53 includes holding section 60 for holding ultrasound probe 101.

Holding section 60 holds ultrasound probe 101 to be located coaxially with third rotation shaft 53. In the present embodiment, holding section 60 includes switch 62 that permits a manual operation of ultrasound probe 101 in a teaching mode described later when an operator manually operates ultrasound probe 101 in a state in which ultrasound probe 101 is held by a hand (holding section 60) of robot arm 21. This may be used as direct teaching enable switch 62 to add a function of stopping robot arm 21 in an emergency.

Robot device 20 of the present embodiment can move ultrasound probe 101 to a certain position in any posture by combining a translational motion in three directions of the X-axis direction, the Y-axis direction, and the Z-axis direction by first arm drive device 35, second arm drive device 36, and lifting and lowering device 40 with a rotational motion in three directions of an X-axis spin (pitching), a Y-axis spin (rolling), and a Z-axis spin (yawing) by rotation three-axis mechanism 50.

Posture holding device 37 holds a posture (an orientation of first rotation shaft 51) of rotation three-axis mechanism 50 in a constant orientation regardless of postures of first arm 22 and second arm 23. Posture holding device 37 includes motor 37a, encoder 37b, and amplifier 37c. A rotation shaft of motor 37a is coupled to posture holding shaft 33 through a decelerator (not illustrated). Posture holding device 37 sets a target rotation angle of posture holding shaft 33 based on a rotation angle of first joint shaft 31 and a rotation angle of second joint shaft 32 such that an axial direction of first rotation shaft 51 is constantly in a left-right direction (the X-axis direction), and drives and controls motor 37a such that posture holding shaft 33 is at a target rotation angle. Thereby, a translational motion in three directions and a rotational motion in three directions can be independently controlled, and thus, control is easily made.

Force sensor 61 is attached to a front end of robot arm 21 and detects a force component acting in each axial direction of an X axis, a Y axis, and a Z axis as an external force acting on robot arm 21 and detects a torque component acting around each axis.

As illustrated in Fig. 3, robot control device 80 includes robot control section 81, monitoring section 82, IO section 83, communication section 84, and storage section 85. Robot control section 81 is configured as a processor including CPU, ROM, RAM, a peripheral circuit, and the like. Monitoring section 82 is configured as a one-chip microcomputer including CPU, ROM, RAM, a peripheral circuit, and the like. Further, monitoring section 82 may be duplicated. Robot control section 81 performs various types of processing related to the control of robot arm 21 (motors 35a to 37a, 44a, and 55a to 57a). Monitoring section 82 monitors states of respective sections, such as IO section 83, communication section 84, amplifiers 35c to 37c, 44c, and 55c to 57c, and sensor sections including encoders 35b to 37b, 44b, and 55b to 57b. IO section 83 is an I/O port, inputs a detection signal from direct teaching enable switch 62, and inputs and outputs a signal from an external device. Communication section 84 performs wired or wireless communication with robot control device 80 and console device 90, and exchanges various control signals and data with each other.

Each of amplifiers 35c to 37c, 44c, and 55c to 57c includes motor control section 71, drive power supply section 72, and IO section 73. Motor control section 71 includes switching elements, and controls respective motors 35a to 37a, 44a, and 55a to 57a by performing switching control of the switching elements based on feedback signals from encoders 35b to 37b, 44b, and 55b to 57b, and the like. Drive power supply section 72 supplies power necessary for driving motors 35a to 37a, 44a, and 55a to 57a. IO section 83 is an I/O port, and inputs various signals, such as position signals from encoders 35b to 37b, 44b, and 55b to 57b, current signals from current sensors that detect currents flowing through respective motors 35a to 37a, 44a, and 55a to 57a, and command signals (control signals) to respective motors 35a to 37a, 44a, and 55a to 57a from robot control section 81.

Robot device 20 has a control-off, control-on, and robot-on as a robot status. The control-off is a state in which robot control section 81 stops the power supply to amplifiers 35c to 37c, 44c, and 55c to 57c such that robot arm 21 (motor) cannot be controlled. The control-on is a state in which robot control section 81 supplies power to amplifiers 35c to 37c, 44c, and 55c to 57c such that robot arm 21 can be controlled. In this state, it is possible to directly touch robot arm 21 to change a posture thereof. The robot-on is a state in which robot control section 81 controls robot arm 21 by outputting control signals to amplifiers 35c to 37c, 44c, and 55c to 57c from the control-on state. The robot status is changed based on an instruction from an operator and changed based on a monitoring result of monitoring section 82.

Next, an operation of robot device 20 provided in ultrasound diagnostic system 10 configured as described above will be described. Robot device 20 of the present embodiment has a direct teaching mode and an automatic operation mode as operation modes.

The direct teaching mode is a mode in which a position and posture of ultrasound probe 101 can be registered at multiple random points while an operator grips and manually operates a hand (holding section 60) of robot arm 21 holding ultrasound probe 101. In the direct teaching mode, robot control section 81 (CPU) obtains position signals from respective encoders 35b to 37b, 44b, and 55b to 57b based on a registration instruction from an operator. Subsequently, robot control section 81 calculates a position and posture of ultrasound probe 101 by forward kinematics based on the obtained position signal. Robot control section 81 stores the calculated position and posture as registration points in storage section 85. A registration instruction of an operator can be executed by, for example, a stepping operation of foot pedal 96, an operation of operation panel 92, an operation by voice recognition, or the like.

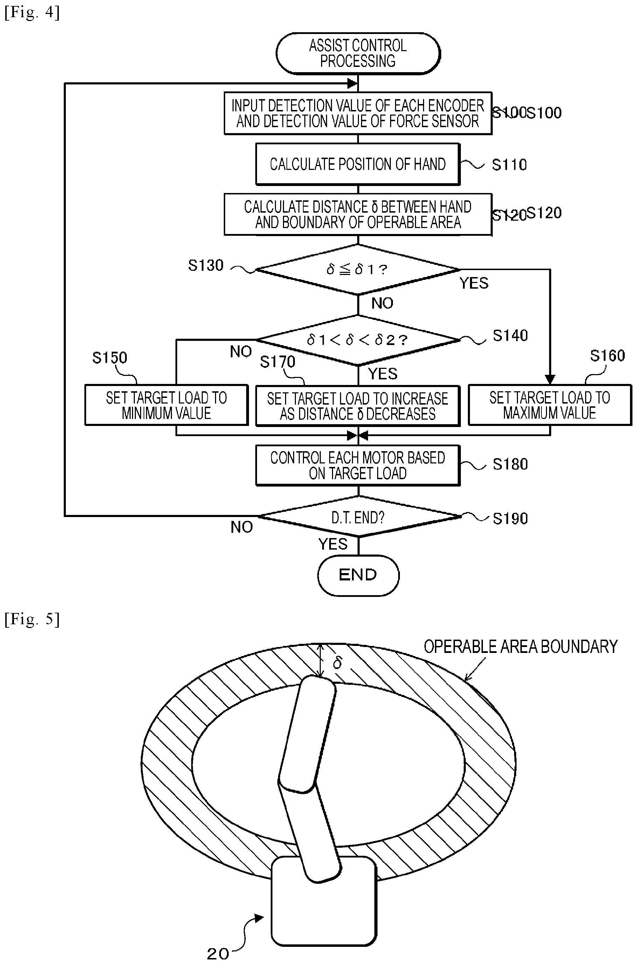

Here, in the direct teaching, assist control for assisting a manual operation of an operator is performed. Fig. 4 is a flowchart illustrating an example of assist control processing performed by robot control section 81. In the assist control processing, robot control section 81 (CPU) first inputs position signals from respective encoders 35b to 37b, 44b, and 55b to 57b and a magnitude and direction of an external force (an operation power operated by an operator) from force sensor 61 (step S100). Subsequently, robot control section 81 calculates a hand position of robot arm 21 by forward kinematics based on the input position signals (step S110). Next, robot control section 81 calculates distance δ between a hand of robot arm 21 and a boundary of an operable area based on the calculated hand position of robot arm 21 (step S 120). Here, as illustrated in Fig. 5, the operable area indicates a range in which robot arm 21 can be appropriately moved by a hand of an operator and is determined around robot device 20. After distance δ is calculated, robot control section 81 determines whether distance δ is equal to or less than first threshold δ1 (step S130) and whether distance δ is greater than first threshold δ1 and less than second threshold δ2 (> δ1) (step S140).

When it is determined that distance δ is equal to or greater than second threshold δ2, robot control section 81 determines that the hand of robot arm 21 is sufficiently separated from a boundary of the operable area and sets a target load, which is a target value of a load applied to the hand of the operator, to the minimum value (step S 150). Robot control section 81 controls respective motors 35a to 37a, 44a, and 55a to 57a such that an assist force corresponding to the set target load acts in a direction of the operation power by the operator (step S180). By minimizing the load applied to the hand of the operator, the operator can operate robot arm 21 with a light load and can smoothly perform a teaching operation.

Meanwhile, when it is determined that distance δ is greater than first threshold δ1 and less than second threshold δ2, robot control section 81 determines that a hand of robot arm 21 is within a diagonal region in Fig. 5 and is close to a boundary of an operable area, and sets a target load to be increased as distance δ is reduced in a range greater than first threshold δ1 and less than second threshold δ2 (step S160). Robot control section 81 controls respective motors 35a to 37a, 44a, and 55a to 57a such that an assist force corresponding to the set target load acts in a direction of the operation power by the operator (step S180). Thereby, since a load applied to a hand of an operator increases as the hand of robot arm 21 approaches the boundary of the operable area, the operator can recognize that the hand of robot arm 21 is approaching the boundary of the operable area.

When it is determined that distance δ is equal to or less than first threshold δ1, robot control section 81 determines that the hand of robot arm 21 reaches the boundary of the operable area, and sets the target load to the maximum value (step S170). Robot control section 81 controls respective motors 35a to 37a, 44a, and 55a to 57a such that an assist force corresponding to the set target load acts in a direction of the operation power by the operator (step S180). In the present embodiment, when the target load is set to the maximum value, a value 0 is set to the assist force. In this case, since no assist force acts on the hand of robot arm 21, performing of the assist control is stopped. Thereby, it is difficult for an operator to operate robot arm 21 out of the operable area.

Next, robot control section 81 determines whether end of direct teaching is requested (step S190). When it is determined that the end of the direct teaching is not requested, robot control section 81 returns to step S100 and repeats the processing of step S100 to step S180. Meanwhile, when it is determined that the end of the direct teaching is requested, robot control section 81 ends the assist control processing.

The automatic operation mode is a mode in which robot device 20 automatically moves ultrasound probe 101 so as to pass through multiple points registered in the direct teaching mode in a predetermined order. In the automatic operation mode, when start of a diagnosis is instructed by an operator, robot control section 81 moves ultrasound probe 101 to a first point among the points registered in advance. The movement of ultrasound probe 101 is performed by determining a target position and a target posture of a hand of robot arm 21, setting a target rotation angle or a target lifting and lowering position of each axis of robot arm 21 by using reverse kinematics from the target position and the target posture, and controlling a corresponding motor such that a rotation angle or a lifting and lowering position detected by each of encoders 35b to 37b, 44b, and 55b to 57b matches a corresponding target rotation angle or target lifting and lowering position. When an operator performs an advance operation, robot control section 81 moves ultrasound probe 101 to a next point. Meanwhile, when the operator performs a return operation, robot control section 81 moves ultrasound probe 101 to an immediately before point. The advance operation or the return operation can be performed by, for example, a stepping operation of foot pedal 96, an operation of operation panel 92, an operation by voice recognition, or the like.

In a case where an ultrasound diagnosis is applied to catheter treatment, when a scan direction of ultrasound probe 101 is taught in advance to match a central axis direction (a length direction) of a blood vessel of an examinee, an operator performs an automatic operation mode and operates foot pedal 96, and thereby, ultrasound probe 101 can be automatically moved in the central axis direction of the blood vessel simply. Thereby, an operator can move a guide wire forward by him/herself while checking a position of a blood vessel of an examinee from an ultrasound echo image by operating ultrasound probe 101.

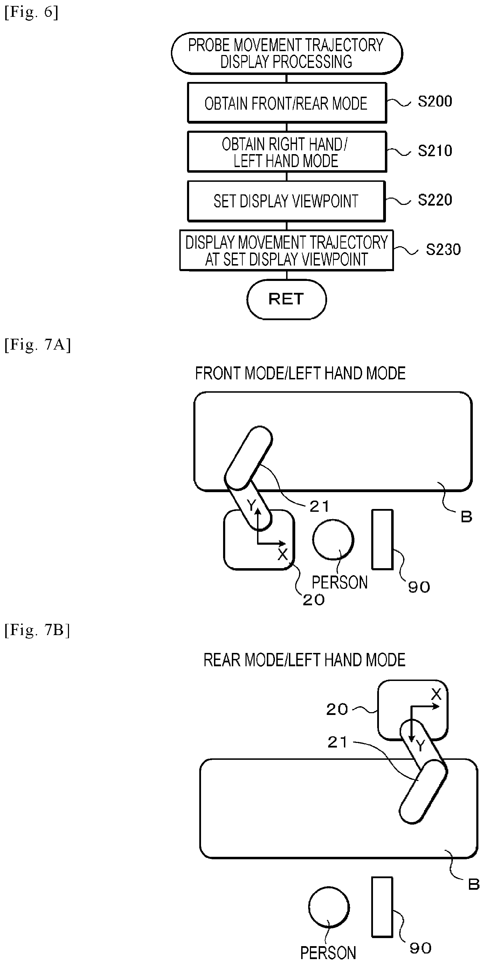

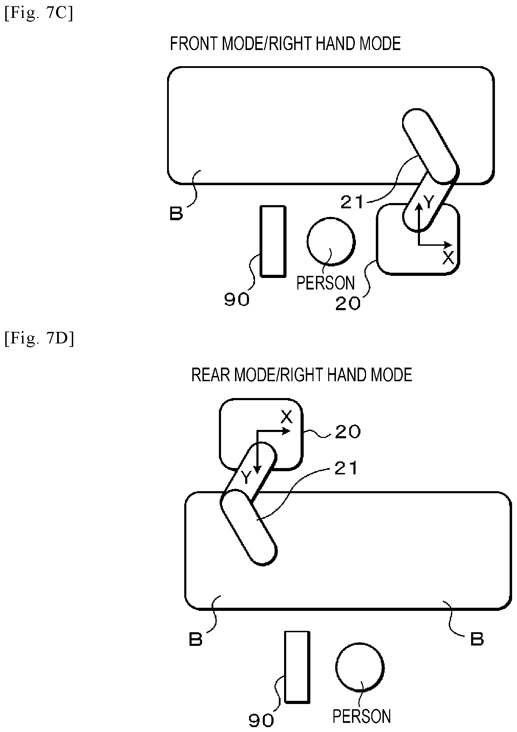

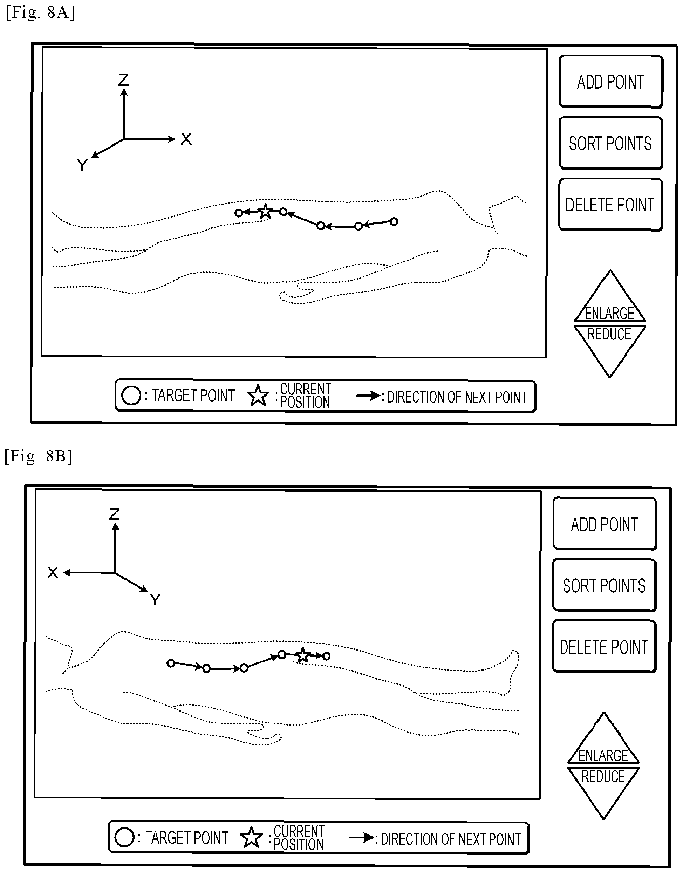

In the automatic operation mode, points (a movement trajectory) registered in the direct teaching mode are three-dimensionally displayed on operation panel 92, and an operator can check a movement destination of ultrasound probe 101 on operation panel 92. Fig. 6 is a flowchart illustrating an example of probe movement trajectory display processing performed by console control section 91 of console device 90. In the probe movement trajectory display processing, console control section 91 first receives selection of either a front mode or a rear mode (step S200), and receives selection of either a left-hand mode or a right-hand mode (step S210). Here, the front mode is a mode in which robot device 20 is installed on the same side as console device 90 with respect to bed B on which an examinee lies. The rear mode is a mode in which robot device 20 is installed on a side opposite to console device 90 with respect to bed B. The left-hand mode is a mode in which robot arm 21 (first arm 22 and second arm 23) is operated by a left-hand system. The right-hand mode is a mode in which robot arm 21 is operated by the right-hand system. Reception of selection of a mode can be performed by an operator performing a touch operation on a selected screen displayed on operation panel 92. Upon receiving the selection of each mode, console control section 91 sets a display aspect based on the received combinations of modes (step S220). The combinations of respective modes include a combination of the front mode and the left-hand mode (see Fig. 7A), a combination of the rear mode and the left-hand mode (see Fig. 7B), a combination of the front mode and the right-hand mode (see Fig. 7C), and a combination of the rear mode and the right-hand mode (see Fig. 7D). The display aspect includes display of a registered point (movement trajectory) in a predetermined coordinate system (for example, world coordinate system) and display of coordinate axes (an X axis, a Y axis, and a Z axis) in the coordinate system. Setting of the display aspect is performed such that a movement direction of the registered point and a direction of an operation of robot arm 21 viewed from an operator are matched to (coincide with) each other. In the present embodiment, when the front mode and left-hand mode are selected and when the rear mode and right-hand mode are selected, the display aspect illustrated in Fig. 8A is set, and when the rear mode and left-hand mode are selected and when the front mode and right-hand mode are selected, the display aspect illustrated in Fig. 8B is set. In the setting of the display aspect, the display of the points (movement trajectory) may be maintained as it is, but only the display of the coordinate axes may be changed according to a positional relationship between robot device 20 and console device 90 or the operation mode of robot arm 21 (the left-hand mode, the right-hand mode). Console control section 91 three-dimensionally displays the points (the movement trajectory of ultrasound probe 101) registered in the set display aspect on operation panel 92 (Step S230), and ends the probe movement trajectory display processing. Thereby, regardless of the positional relationship between robot device 20 and console device 90 and the operation mode (the left-hand mode, the right-hand mode) of robot arm 21, the points (movement trajectory) registered in the direct teaching mode can be displayed in an aspect which can be easily confirmed by an operator. In the automatic operation mode, the operator can operate foot pedal 96 when the hand is full, and thereby, a reproduction operation of ultrasound probe 101 can be performed.

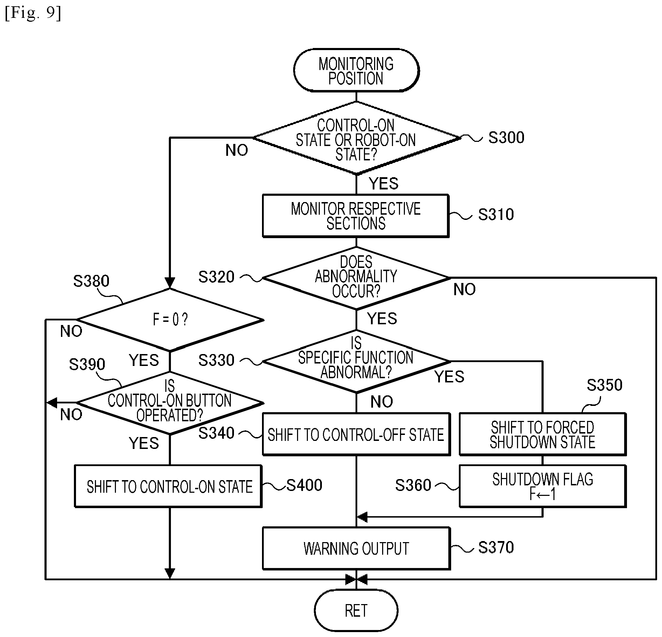

Next, an operation of monitoring section 82 of robot control device 80 will be described. Fig. 9 is a flowchart illustrating an example of monitoring performed by monitoring section 82. This processing is repeatedly performed every predetermined time period.

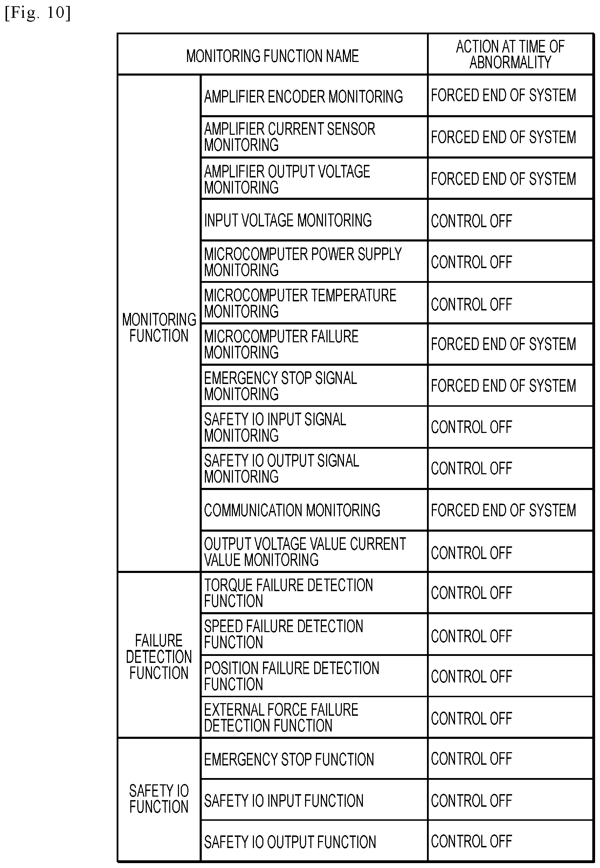

In the monitoring, monitoring section 82 (CPU) first determines whether a current robot status is a control-on state or a robot-on state (step S300). When it is determined that the current robot status is a control-on state or the robot-on state, monitoring section 82 monitors respective sections constituting robot device 20, such as IO section 83, communication section 84, amplifiers 35c to 37c, 44c, and 55c to 57c, and a sensor section (step S310). As illustrated in Fig. 10, functions of monitoring section 82 include a monitoring function, a failure detection function, and a safety IO function.

The monitoring function includes amplifier encoder monitoring, amplifier current sensor monitoring, amplifier output voltage monitoring, input voltage monitoring, microcomputer power supply start, microcomputer temperature monitoring, microcomputer failure monitoring, emergency stop signal monitoring, safety IO input signal monitoring, safety IO output signal monitoring, communication monitoring, and output voltage value current value monitoring. The amplifier encoder monitoring is for determining failures of encoders 35b to 37b, 44b, and 55b to 57b. The amplifier current sensor monitoring is for determining a failure of a current sensor that detects an output current to motors 35a to 37a, 44a, and 55a to 57a. The amplifier output voltage monitoring is for determining a failure of motor control section 71 (switching element). The input voltage monitoring is for monitoring a voltage supplied to robot control device 80 (main board). The microcomputer power supply start is for monitoring a voltage supplied to a microcomputer (robot control section 81 and monitoring section 82). The microcomputer temperature monitoring is for monitoring an ambient temperature of a microcomputer. The microcomputer failure monitoring is for determining a failure of a microcomputer. The emergency stop signal monitoring is for determining a failure of emergency stop switch 95. The safety IO input signal monitoring is for monitoring an input signal input to control section 81 other than the emergency stop signal. The safety IO output signal monitoring is for monitoring an output signal output from control section 80. The communication monitoring is for monitoring communication of communication section 84. The output voltage value current value monitoring is for monitoring a voltage value and a current value output from robot control device 80 (robot control section 81) to amplifiers 35c to 37c, 44c, and 55c to 57c.

The failure detection function includes a torque failure detection function, a speed failure detection function, a position failure detection function, and an external force failure detection function. The torque failure detection function is for detecting a state in which torque exceeding a set torque is output to each axis or hand of robot arm 21. The speed failure detection function is for detecting a state in which each axis or hand of robot arm 21 operates at a speed exceeding a set speed. The position failure detection function is for detecting a state in which each axis or hand of robot arm 21 is located outside a set range. The external force failure detection function is for detecting a state in which an external force exceeding an allowable range is applied to each axis or hand of robot arm 21.

The safety IO function includes an emergency stop function, a safety IO input function, and a safety IO output function. The emergency stop function is for detecting an ON operation of emergency stop switch 95. The safety IO input function is for detecting a state in which joint controller 97 operates beyond a set operation range when robot arm 21 operates by joint controller 97. The safety IO output function is for detecting a state in which joint controller 97 operates at a speed exceeding a set maximum speed. Further, the safety IO output function is effective when coupling a device (for example, a laser curtain or another robot) which is not provided in the present device.

When monitoring respective sections constituting the system in this manner, monitoring section 82 determines whether an abnormality occurs in any of the respective sections (step S320). When determining that no abnormality occurs in any of the respective sections, monitoring section 82 ends the monitoring. Meanwhile, when it is determined that an abnormality occurs in any of the respective sections, monitoring section 82 determines whether the occurred abnormality is an abnormality (specific abnormality) of a specific type of a specific portion which is an important element for proper operation of a system (step S330). In the present embodiment, the specific abnormality includes respective abnormalities related to amplifier encoder monitoring, amplifier current sensor monitoring, amplifier output voltage monitoring, microcomputer failure monitoring, emergency stop signal monitoring, and communication monitoring. When it is determined that the occurred abnormality is not the specific abnormality, monitoring section 82 shifts the robot status to the control-off state (step S340), outputs a warning to operation panel 113 or speaker 114 (S370), and ends the monitoring. Meanwhile, when it is determined that the occurred abnormality is the specific abnormality, monitoring section 82 shifts the robot status to a forced shutdown state (Step S350), sets shutdown flag F to a value 1 (Step S360), outputs a warning to operation panel 113 or speaker 114 (S370), and ends the monitoring. Shutdown flag F is set to a value 0 by initialization processing when supplying (time of starting) power to ultrasound diagnostic system 10, and is set to a value 1 when shifting to the forced shutdown state. An output of the warning is performed as monitoring section 82 outputs a warning signal including an occurrence location and type of the abnormality, the shifted robot status, and the like to console device 90 through communication. Thereby, an operator checks warning content and performs work of repair or maintenance as necessary.

When the monitoring is performed after shifting to the control-off state or the forced shutdown state, it is not determined to be in the control-on state or the robot-on state in step S300, and accordingly, monitoring section 82 then determines whether a shutdown flag is the value 0 (step S380). When it is determined that the shutdown flag is the value 0, whether a control-on button provided in console device 90 is operated is determined (step S390). When it is determined that the control-on button is operated, monitoring section 82 is shifted (returns) to the control-on state (step S400), and the monitoring ends. That is, when shifting to the control-off state is made due to any abnormality, an operator can operate the control-on button, and thereby, the robot status can return to the control-on state. However, when the robot status is returned to the control-on state without resolving an abnormality, the abnormality is detected again, and the robot status will be shifted to the control-off state.

Meanwhile, when it is determined that shutdown flag F is not the value 0 but the value 1 or when it is determined that the control-on button is not operated, monitoring section 82 is not shifted (returned) to the control-on state, and the monitoring ends. In the forced shutdown state, shutdown flag F is set to the value 0 by shutting down and starting a power supply of a system, and accordingly, it is possible to return to the control-on state by operating the control-on button. As described above, the forced shutdown state is common to the control-off state in that the power supply to amplifiers 35c to 37c, 44c, and 55c to 57c stops, and differs from the control-off state, which does not require a shutdown to return to the control-on state, in that the shutdown of a system is required to return to the control-on state. Thereby, the safety of robot device 20 can be guaranteed by shifting to a different state depending on a location of abnormality or the type of abnormality. In the present embodiment, a safety state that is shifted due to an abnormality of a system includes a control-off state and a forced shutdown state, and monitoring section 82 is shifted to a different safety state depending on a location of abnormal and the type of abnormality but may be shifted to the control-off state or to the forced shutdown state regardless of the abnormality location or the type of abnormality.

Here, a correspondence relationship between main elements of the embodiment and main elements of the present disclosure described in the claims will be described. That is, ultrasound probe 101 of the present embodiment corresponds to an ultrasound probe of the present disclosure, robot arm 21 corresponds to an arm section, motors 35a to 37a, 44a, and 55a to 57a correspond to a motor section, amplifiers 35c to 37c, 44c, and 55c to 57c correspond to an amplifier section, robot control section 81 corresponds to a robot control section, and monitoring section 82 corresponds to a monitoring section. Further, operation panel 92 corresponds to a display section.

It is needless to say that the present disclosure is not limited to the embodiment described above in any way, and hence can be practiced in various ways as long as it falls within the technical scope of the present disclosure.

For example, in the embodiments described above, robot device 20 is configured as a seven-axes articulated robot capable of performing a translational motion in three directions and a rotational motion in three directions. However, the number of axes may be any number. Further, robot device 20 may be configured with a so-called vertical articulated robot, a horizontal articulated robot, or the like.

As described above, the ultrasound diagnostic system of the present disclosure monitors states of respective sections constituting a system including an arm section, a motor section, an amplifier section, and a robot control section, and is shifted to a predetermined safety state when an abnormality occurs in any of the respective sections. Thereby, it is possible to perform ultrasound diagnosis while ensuring safety.

In the ultrasound diagnostic system of the present disclosure described above, a status of the robot may include an ON state in which a power supply of the amplifier section is turned on and an OFF state in which the power supply of the amplifier section is turned off, and the monitoring section may be shifted to the OFF state as the predetermined safety state when an abnormality occurs in any one of the respective sections in the ON state. By turning off the power supply of the amplifier section due to occurrence of an abnormality, an unexpected operation can be prevented more reliably from occurring in a robot.

Further, in the ultrasound diagnostic system of the present disclosure, the safety state may include multiple safety states, and when an abnormality occurs in any one of the respective sections, the monitoring section may determine a state that is shifted from among the multiple safety states depending on an abnormality occurrence location or the type of abnormality. Accordingly, high safety can be ensured even when any abnormality occurs in a system. In this case, a status of the robot may include an ON state in which a power supply of the amplifier section is turned on, a first OFF state in which the power supply of the amplifier section is turned off and returnable to the ON state by a predetermined release operation, and a second OFF state in which the power supply of the amplifier section is turned off and returnable to the ON state by shutting down and starting a power supply of a system, and when an abnormality occurs in any of the respective sections, the monitoring section may determine a state to be shifted among the first OFF state and the second OFF state depending on an occurrence location of the abnormality or the type of abnormality.

Furthermore, in the ultrasound diagnostic system of the present disclosure, an operation mode of the robot may include a direct teaching mode in which an operator manually operates the arm section within a predetermined operable range to record a movement trajectory of the arm section, and the robot control section may output, to the amplifier section, a control signal for driving and controlling the motor section such that a load applied to an operator increases as the operator manually operates the arm section as the arm section approaches a boundary of the operable range in the direct teaching mode. Accordingly, even when an operator does not confirm an operable range in advance, the operator can recognize that a boundary of the operable range is approached by a load applied to a hand during a manual operation, and direct teaching can be appropriately performed.

Further, the ultrasound diagnostic system of the present disclosure may further include a display section configured separately from a robot main body including the arm section and the motor section, and the robot control section may output, to the amplifier section, a control signal for driving the motor section such that the ultrasound probe held by the arm section moves according to a movement trajectory recorded in advance, and the display section may display the movement trajectory and change a display aspect of the movement trajectory based on an installation position of the robot main body. Accordingly, even when a positional relationship between a robot main body and a display section is changed, an operator can check a movement trajectory of an ultrasound probe in an easily viewable aspect. The arm section may include a horizontal joint arm, an operation mode of the arm section may include a left-hand mode in which the arm section is operated by a left-hand system and a right-hand mode in which the arm section is operated by a right-hand system, and the display section may change a display viewpoint of the movement trajectory based on the operation mode.

The present disclosure is not limited to a form of an ultrasound diagnostic system and can be a form of a monitoring method for the ultrasound diagnostic system.

### Industrial Applicability

The present disclosure is applicable to an ultrasound diagnostic device, a manufacturing industry of a robot, and the like.

### Reference Signs List

10 ultrasound diagnostic system, 20 robot device, 21 robot arm, 22 first arm, 23 second arm, 24 base, 25 base plate, 26 caster, 27 lever, 28 lock section, 31 first joint shaft, 32 second joint shaft, 33 posture holding shaft, 35 first arm drive device, 35a motor, 35b encoder, 35c amplifier, 36 second arm drive device, 36a motor, 36b encoder, 36c amplifier, 37 posture holding device, 37a motor, 37b encoder, 37c amplifier, 40 lifting and lowering device, 41 first slider, 42 first guide member, 43 first ball screw shaft, 44a motor, 44b encoder, 44c amplifier, 45 height adjustment mechanism, 46 second slider, 47 second guide member, 48 second ball screw shaft, 49 operation handle, 50 rotation three-axis mechanism, 51 first rotation shaft, 52 second rotation shaft, 53 third rotation shaft, 55 first rotation device, 55a motor, 55b encoder, 55c amplifier, 56 second rotation device, 56a motor, 56b encoder, 56c amplifier, 57 third rotation device, 57a motor, 57b encoder, 57c amplifier, 60 holding section, 61 force sensor, 62 direct teaching enable switch, 71 motor control section, 72 drive power supply section, 73 IO section, 80 robot control device, 81 robot control section, 82 monitoring section, 83 IO section, 84 communication section, 85 storage section, 90 console device, 91 console control section, 92 operation panel, 93 speaker, 94 communication section, 95 emergency stop switch, 96 foot pedal, 97 joint controller, 100 ultrasound diagnostic device, 101 ultrasound probe, 102 cable, 110 ultrasound diagnostic device main body, 111 ultrasound diagnostic control section, 112 image processing section, 113 image display section

## Claims

1. An ultrasound diagnostic system for performing diagnosis by moving an ultrasound probe by a robot, comprising:
an arm section configured to hold the ultrasound probe;
a motor section configured to move the arm section;
an amplifier section configured to supply power to the motor section;
a robot control section configured to output a control signal to the amplifier section; and
a monitoring section configured to monitor states of respective sections constituting a system and shift to a predetermined safety state when an abnormality occurs in any of the respective sections.

2. The ultrasound diagnostic system according to Claim 1, wherein
a status of the robot includes an ON state in which a power supply of the amplifier section is turned on and an OFF state in which the power supply of the amplifier section is turned off, and
the monitoring section shifts to the OFF state as the predetermined safety state when an abnormality occurs in any one of the respective sections in the ON state.

3. The ultrasound diagnostic system according to Claim 1, wherein
the safety state includes multiple safety states, and
when an abnormality occurs in any one of the respective sections, the monitoring section determines a state to be shifted among the multiple safety states depending on an occurrence location of the abnormality or a type of the abnormality.

4. The ultrasound diagnostic system according to Claim 3, wherein
a status of the robot includes an ON state in which a power supply of the amplifier section is turned on, a first OFF state in which the power supply of the amplifier section is turned off and returnable to the ON state by a predetermined release operation, and a second OFF state in which the power supply of the amplifier section is turned off and returnable to the ON state by shutting down and starting a system power supply, and
when an abnormality occurs in any of the respective sections, the monitoring section determines a state to be shifted among the first OFF state and the second OFF state depending on an occurrence location of the abnormality or a type of the abnormality.

5. The ultrasound diagnostic system according to any one of Claims 1 to 4, wherein
an operation mode of the robot includes a direct teaching mode in which an operator manually operates the arm section within a predetermined operable range to record a movement trajectory of the arm section, and
the robot control section outputs, to the amplifier section, a control signal for driving and controlling the motor section such that a load applied to an operator increases as the operator manually operates the arm section as the arm section approaches a boundary of the operable range in the direct teaching mode.

6. The ultrasound diagnostic system according to any one of Claims 1 to 5, further comprising:
a display section configured separately from a robot main body including the arm section and the motor section,
wherein the robot control section outputs, to the amplifier section, a control signal for driving the motor section such that the ultrasound probe held by the arm section moves according to a movement trajectory recorded in advance, and
the display section displays the movement trajectory and changes a display aspect of the movement trajectory based on an installation position of the robot main body.

7. A monitoring method for an ultrasound diagnostic system that includes an arm section configured to hold an ultrasound probe, a motor section configured to move the arm section, an amplifier section configured to supply power to the motor section, and a robot control section configured to output a control signal to the amplifier section and performs diagnosis by moving the ultrasound probe by a robot, the monitoring method comprising:
monitoring states of respective sections constituting a system; and
shifting to a predetermined safety state when an abnormality occurs in any one of the respective sections.
